# EUROPÄISCHE PATENTSCHRIFT

(11) **EP 3 355 963 B1**
(45) Veröffentlichungstag und Bekanntmachung des Hinweises auf die Patenterteilung: **17.02.2021**
(21) Anmeldenummer: 16770684.5
(22) Anmeldetag: 26.09.2016
(51) Int. Cl.: A61M 5/20, A61M 5/315, A61M 5/48, F16D 63/00

(54) **INJEKTIONSGERÄT**
INJECTION APPARATUS
DISPOSITIF D'INJECTION

(30) Priorität: 30.09.2015 DE 202015006842 U
(43) Veröffentlichungstag der Anmeldung: 08.08.2018
(73) Patentinhaber: Haselmeier AG, 9001 St. Gallen (CH)
(72) Erfinder: KEITEL, Joachim, 73728 Esslingen (DE)
(74) Vertreter: Manitz Finsterwald Patent- und Rechtsanwaltspartnerschaft mbB
(86) Internationale Anmeldenummer: PCT/EP2016/001598
(87) Internationale Veröffentlichungsnummer: WO 2017/054915

(56) Entgegenhaltungen:
- WO-A1-2010/000085
- WO-A1-2014/166900
- WO-A1-2014/166918
- WO-A1-2015/091766
- DE-A1-102005 060 928
- DE-A1-102007 013 836
- DE-U1-202014 001 136

## Beschreibung

Die Erfindung betrifft ein Injektionsgerät der im Oberbegriff des Anspruchs 1 angegebenen Gattung.

Aus der WO 2014/166918 A1 geht ein Injektionsgerät hervor, bei dem die Injektionsgeschwindigkeit einstellbar ist. Das Injektionsgerät besitzt ein Antriebsteil, das sich beim Auspressen von Injektionsflüssigkeit dreht und dadurch eine Kolbenstange in axialer Richtung bewegt. Zur Einstellung der Injektionsgeschwindigkeit ist ein Reibungselement vorgesehen, das das Antriebsteil beim Injektionsvorgang bremst. Das Reibungselement ist axial und in Umfangsrichtung ortsfest im Gehäuse des Injektionsgeräts gehalten. Die Injektionsgeschwindigkeit wird durch Verschieben des Antriebsteils in axialer Richtung eingestellt.

Die DE 10 2007 013 836 A1 offenbart eine Injektionsvorrichtung mit gesteuertem Nadelrückzug. Das Injektionsgerät besitzt eine Rastvorrichtung, die beim Auspressen von Injektionsflüssigkeit Klicksignale abgibt.

Die DE 20 2014 001 136 U1 offenbart ein Injektionsgerät, bei dem eine Rasteinrichtung zwischen einem Einstellteil und dem Gehäuse wirkt, die beim Einstellen einer aus dem Behälter auszupressenden Menge an Injektionsflüssigkeit wirksam ist.

Aus der WO 2014/166900 A1 geht ein automatisches Injektionsgerät hervor, bei dem zur Einstellung der Injektionsgeschwindigkeit eine Hülse vorgesehen ist. Die Hülse dreht sich bei Betätigung des Auspressknopfes und verändert dabei die freie Länge eines Rastelements. Dadurch kann die Injektionsgeschwindigkeit eingestellt werden.

Die DE 10 2005 060 928 A1 offenbart ein Injektionsgerät mit Rastvorrichtungen.

Die WO 2010/000085 A1 offenbart eine Bremsvorrichtung mit gefederten Stiften, deren radiale Position gegenüber einer zu bremsenden Welle einstellbar ist.

Der Erfindung liegt die Aufgabe zugrunde, ein Injektionsgerät der gattungsgemäßen Art zu schaffen, das einen einfachen Aufbau und eine sichere Funktion besitzt.

Diese Aufgabe wird durch ein Injektionsgerät mit den Merkmalen des Anspruchs 1 gelöst.

Das Injektionsgerät besitzt eine Einstellvorrichtung zur Einstellung der Injektionsgeschwindigkeit, mit der die Injektionsflüssigkeit aus dem Behälter ausgepresst wird. Dadurch kann der Bediener eine für ihn angenehme und beispielsweise auf den Durchmesser der verwendeten Nadel und die Viskosität der Flüssigkeit im Behälter abgestimmte Injektionsgeschwindigkeit einstellen. Die Einstellvorrichtung beeinflusst dabei die Energie, die zur Bewegung des zweiten Bauteils gegenüber dem ersten Bauteil benötigt wird. Bei hoher benötigter Energie verläuft die Injektion vergleichsweise langsam, während die Injektion bei geringer benötigter Energie vergleichsweise schnell verläuft. Um einen einfachen Aufbau des Injektionsgeräts zu erreichen, ist vorgesehen, dass das erste Bauteil beim Auspressen von Injektionsflüssigkeit in Richtung der Längsmittelachse beweglich und drehfest im Gehäuse geführt ist. Über die axiale Position des ersten Bauteils kann die gewünschte Injektionsgeschwindigkeit dadurch auf einfache Weise eingestellt werden. Das zweite Bauteil ist beim Auspressen von Injektionsflüssigkeit aus dem Behälter drehbar und in Richtung der Längsmittelachse ortsfest gegenüber dem Gehäuse gelagert. Die Bewegungen zum Einstellen der Injektionsgeschwindigkeit und zum Auspressen der Injektionsflüssigkeit können dadurch durch voneinander getrennte Bewegungen des ersten Bauteils und des zweiten Bauteils ausgeführt werden. Dadurch wird ein einfacher Aufbau erreicht.

Vorteilhaft ermöglicht die Einstellvorrichtung eine stufenlose Einstellung der Injektionsgeschwindigkeit. Dadurch ergibt sich eine besonders ergonomische Handhabung. Es kann jedoch auch vorgesehen sein, dass die Einstellvorrichtung unterschiedliche einstellbare Geschwindigkeitsstufen vorgibt. Ein einfacher Aufbau bei geringem Bauraum der Einstellvorrichtung wird dadurch erreicht, dass die Einstellvorrichtung bei der Einstellung der Injektionsgeschwindigkeit auf die axiale Relativposition von erstem Bauteil und zweitem Bauteil wirkt.

Die Einstellvorrichtung umfasst vorteilhaft ein Bedienelement, das von einer Einstellfeder vorgespannt ist. Die Einstellfeder wirkt dabei einer Bewegung des Bedienelements in Richtung auf eine Erhöhung der Injektionsgeschwindigkeit entgegen. Aufgrund der Einstellfeder wird das Bedienelement während der Injektion in Richtung auf die niedrigste einstellbare Injektionsgeschwindigkeit gedrückt. Die niedrigste einstellbare Injektionsgeschwindigkeit kann dabei auch eine Geschwindigkeit von 0 sein. Die von der Einstellfeder ausgeübte Kraft muss der Bediener bei der Injektion permanent überwinden, um eine höhere als die niedrigste einstellbare Injektionsgeschwindigkeit zu erreichen. Dadurch ist eine dynamische Einstellung der Injektionsgeschwindigkeit während des Einstellvorgangs möglich. Ist die niedrigste einstellbare Injektionsgeschwindigkeit 0, so kann der Bediener die Injektion durch Loslassen des Bedienelements unterbrechen und durch erneute Betätigung des Bedienelements fortsetzen.

Das Bedienelement bildet vorteilhaft das erste Bauteil und ist zum Auspressen von Injektionsflüssigkeit entgegen der von der Einstellfeder ausgeübten Kraft in proximaler Richtung zu verschieben. Die Einstellfeder wirkt dabei bevorzugt zwischen dem Bedienelement und dem Gehäuse. In vorteilhafter Ausgestaltung stützt sich die Einstellfeder mit einem Ende am Bedienelement und mit dem anderen Ende am Gehäuse ab. Die zur Einstellung der Injektionsgeschwindigkeit benötigte Kraft kann dadurch, dass die Einstellfeder unmittelbar zwischen dem Bedienelement und dem Gehäuse wirkt und das Bedienelement ausschließlich in axialer Richtung beweglich ist, präzise und auf einfache Weise durch geeignete Auslegung der Einstellfeder eingestellt werden.

Zur Beeinflussung der Energie, die zur Bewegung des ersten Bauteils gegenüber dem zweiten Bauteil benötigt wird, ist eine Rasteinrichtung vorgesehen, die zwischen dem ersten und dem zweiten Bauteil wirkt. Die Rasteinrichtung beeinflusst dabei vorteilhaft das Drehmoment, das zur Drehung des zweiten Bauteils gegenüber dem ersten Bauteil benötigt wird. Die Rasteinrichtung benötigt dabei in einer ersten Relativposition von erstem Bauteil und zweitem Bauteil eine höhere Energie, insbesondere ein höheres Drehmoment, zum Überwinden einer Rastung als in einer zweiten Relativposition von erstem Bauteil und zweitem Bauteil. Es kann auch vorgesehen sein, dass die Einstellvorrichtung zusätzlich zu einer Rasteinrichtung eine Reibkraft beeinflusst, die zwischen dem ersten Bauteil und dem zweiten Bauteil wirkt. Über die Rasteinrichtung können vergleichsweise große Kräfte zwischen dem ersten Bauteil und dem zweiten Bauteil aufgebracht werden. Dadurch kann die Injektionsfeder vergleichsweise stark ausgelegt werden, so dass auch bei hoher Viskosität der auszupressenden Flüssigkeit und sehr dünner verwendeter Nadel ein sicheres Auspressen von Injektionsflüssigkeit erreicht wird. Bei sehr geringer gewünschter Injektionsgeschwindigkeit kann auch bei stark ausgelegter Injektionsfeder eine ausreichend große Kraft zwischen dem ersten Bauteil und dem zweiten Bauteil erzeugt werden, so dass eine geringe Injektionsgeschwindigkeit einstellbar ist.

Ein einfacher Aufbau ergibt sich, wenn die Rasteinrichtung mindestens ein Rastelement umfasst, das mit mindestens einem Gegenrastelement zusammenwirkt, wobei die Rasttiefe, mit der die Rastelemente und die Gegenrastelemente sich überlappen, in der ersten Relativposition größer als in der zweiten Relativposition ist. Um eine stufenlose Verstellung der Einstellvorrichtung zu ermöglichen, ist vorteilhaft vorgesehen, dass das Rastelement in einer Schnittdarstellung, die die Längsmittelachse enthält, eine zur Längsmittelachse geneigt verlaufende Rastkante besitzt und dass das Gegenrastelement eine zur Längsmittelachse geneigt verlaufende Gegenrastkante besitzt. Die Neigungswinkel von Rastkante und Gegenrastkante sind dabei vorteilhaft gleich groß. Die Neigungswinkel können beispielsweise von 1° bis 20°, insbesondere von 2° bis 10° betragen. Die Neigungswinkel sind abhängig von dem Weg, den die Rastelemente zwischen der kleinsten und der größten einstellbaren Geschwindigkeit relativ zueinander zurücklegen sollen und von der Differenz zwischen der kleinsten und der größten Geschwindigkeit. Der Neigungswinkel ist auch abhängig von der Gestaltung der Rasteinrichtung, nämlich von der Geometrie der Verrastung und der Steifigkeit der verwendeten Materialien für Rastelement und Gegenrastelement. Der Neigungswinkel kann anhand der gewünschten Auslegung geeignet gewählt werden.

Ein einfacher Aufbau ergibt sich, wenn das erste Rastelement als Raststeg ausgebildet ist, der in eine das Gegenrastelement bildende Rastvertiefung ragt. Um eine gleichmäßige Injektionsgeschwindigkeit zu erreichen, ist bevorzugt eine Vielzahl von Rastelementen und von Gegenrastelementen vorgesehen. Vorteilhaft ist die Anzahl von als Raststeg ausgebildeten Rastelementen größer als die von als Gegenrastelement ausgebildeten Rastvertiefungen.

Vorteilhaft sind das erste Bauteil und das zweite Bauteil beim Einstellen einer auszupressenden Menge an Injektionsflüssigkeit drehfest miteinander verbunden. Dann kann die Einstellvorrichtung beim Einstellen einer auszupressenden Menge an Injektionsflüssigkeit in irgendeiner Stellung angeordnet sein, insbesondere in einer Stellung, in der die Einstellvorrichtung eine freie Drehung der Bauteile zueinander zulässt. Ein einfacher Aufbau ergibt sich, wenn mindestens ein Rastelement der Rasteinrichtung Teil einer Kupplung zwischen dem ersten Bauteil und dem zweiten Bauteil ist, die das erste Bauteil und das zweite Bauteil beim Einstellen einer auszupressenden Menge an Injektionsflüssigkeit drehfest verbindet. Beim Einstellen der auszupressenden Menge an Injektionsflüssigkeit dient das mindestens eine Rastelement dadurch zur drehfesten Verbindung der Bauteile. Beim Auspressen der auszupressenden Menge an Injektionsflüssigkeit dient das Rastelement zur Einstellung des Drehmoments, das zwischen erstem Bauteil und zweitem Bauteil bei einer Relativdrehung der Kraft der Injektionsfeder entgegenwirkt.

Ein einfacher Aufbau des Injektionsgeräts ergibt sich, wenn das Injektionsgerät eine Injektionshülse besitzt, die sich beim Einstellen einer Dosis von Injektionsflüssigkeit in Richtung der Längsmittelachse gegenüber dem Gehäuse bewegt, und die Injektionsfeder als Schraubendruckfeder ausgebildet ist, die sich mit einem ersten Ende gegenüber der Injektionshülse und mit einem zweiten Ende gegenüber dem Gehäuse abstützt. Die Injektionsfeder muss sich dabei nicht unmittelbar an der Injektionshülse oder am Gehäuse abstützen, sondern kann auch an Bauteilen abgestützt sein, die eine entsprechende axiale Relativbewegung ausführen. Vorteilhaft legt die Injektionshülse die auszupressende Menge an Injektionsflüssigkeit fest, wobei die Injektionshülse zur Einstellung der auszupressenden Menge an Injektionsflüssigkeit vorteilhaft in distaler Richtung bewegt wird und die proximale Endlage der Injektionshülse das Injektionsende festlegt.

Ein Ausführungsbeispiel der Erfindung wird im Folgenden anhand der Zeichnung erläutert. Es zeigen:
- Fig. 1: eine Seitenansicht eines Injektionsgeräts in Nullstellung,
- Fig. 2: einen Schnitt entlang der Linie II-II in Fig. 1,
- Fig. 3: eine Seitenansicht des Injektionsgeräts aus Fig. 1 in Maximalstellung,
- Fig. 4: einen Schnitt entlang der Linie IV-IV in Fig. 1,
- Fig. 5: eine perspektivische Darstellung des Bedienelements des Injektionsgeräts aus den Fig. 1 bis 4,
- Fig. 6: eine Seitenansicht des Bedienelements aus Fig. 5,
- Fig. 7: einen Schnitt entlang der Linie VII-VII in Fig. 6,
- Fig. 8: eine Ansicht des Bedienelements in Richtung des Pfeils VIII in Fig. 6,
- Fig. 9: eine schematische Darstellung eines Raststegs des Bedienelements,
- Fig. 10: eine schematische perspektivische Darstellung des Bedienelements in zwei Einzelteilen,
- Fig. 11: eine Seitenansicht der beiden Einzelteile des Bedienelements,
- Fig. 12: eine Seitenansicht des Mitnehmers des Injektionsgeräts,
- Fig. 13: einen Schnitt entlang der Linie XIII-XIII in Fig. 12,
- Fig. 14: einen Schnitt entlang der Linie XIV-XIV in Fig. 12,
- Fig. 15: einen Schnitt entlang der Linie XV-XV in Fig. 12,
- Fig. 16: einen Schnitt entlang der Linie XVI-XVI in Fig. 12,
- Fig. 17: eine Seitenansicht des Verbindungselements des Injektionsgeräts,
- Fig. 18: einen Schnitt entlang der Linie XVIII-XVIII in Fig. 17,
- Fig. 19: eine Seitenansicht einer Kolbenstange des Injektionsgeräts,
- Fig. 20: einen Schnitt entlang der Linie XX-XX in Fig. 19,
- Fig. 21: einen Schnitt entlang der Linie XXI-XXI in Fig. 19,
- Fig. 22: eine Seitenansicht von Bedienelement und Mitnehmer in einer Kupplungsposition,
- Fig. 23: einen Schnitt entlang der Linie XXIII-XXIII in Fig. 22,
- Fig. 24: eine Seitenansicht von Bedienelement und Mitnehmer in einer ersten Relativposition,
- Fig. 25: einen Schnitt entlang der Linie XXV-XXV in Fig. 24,
- Fig. 26: eine Seitenansicht von Bedienelement und Mitnehmer in einer zweiten Relativposition,
- Fig. 27: einen Schnitt entlang der Linie XXVII-XXVII in Fig. 26,
- Fig. 28: eine Seitenansicht von Bedienelement und Mitnehmer in einer dritten Relativposition,
- Fig. 29: einen Schnitt entlang der Linie XIX-XIX in Fig. 28,
- Fig. 30: eine Seitenansicht eines Dosierorgans des Injektionsgeräts,
- Fig. 31: einen Schnitt entlang der Linie XXXI-XXXI in Fig. 30,
- Fig. 32: eine Seitenansicht einer Injektionshülse des Injektionsgeräts,
- Fig. 33: einen Schnitt entlang der Linie XXXIII-XXXIII in Fig. 32,
- Fig. 34: einen Schnitt entlang der Linie XXXIV-XXXIV in Fig. 32,
- Fig. 35: eine Seitenansicht eines oberen Gehäuseteils des Injektionsgeräts,
- Fig. 36: einen Schnitt entlang der Linie XXXVI-XXXVI in Fig. 35,
- Fig. 37: einen Schnitt entlang der Linie XXXVII-XXXVII in Fig. 35,
- Fig. 38: eine Seitenansicht in Richtung des Pfeils XXXVIII in Fig. 36.

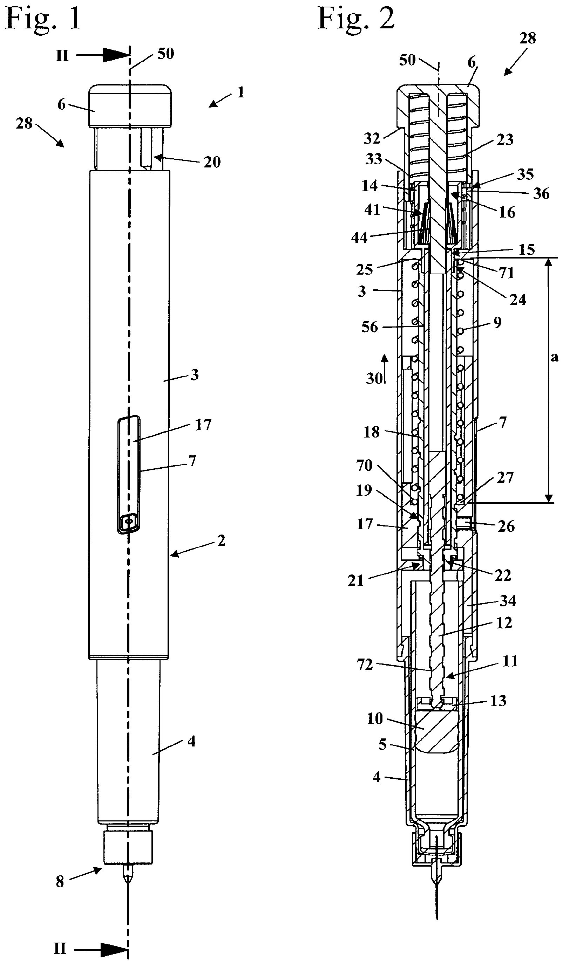

Fig. 1 zeigt als Ausführungsbeispiel für ein mechanisches Injektionsgerät, bei dem das Auspressen einer Dosis von Injektionsflüssigkeit automatisch erfolgt, ein Injektionsgerät 1. Das Injektionsgerät 1 besitzt ein Gehäuse 2, das ein oberes Gehäuseteil 3 und einen am oberen Gehäuseteil 3 festgelegten Halter 4 umfasst. Der Halter 4 ist an der proximalen Seite des oberen Gehäuseteils 3 angeordnet. An der proximalen Seite des Halters 4 ist eine Injektionsnadel 8 festgelegt. An der distalen Seite des Injektionsgeräts 1 ist ein Bedienelement 6 angeordnet. Das Bedienelement 6 ist über eine Kupplung 20 mit dem oberen Gehäuseteil 3 drehfest verbindbar. Das obere Gehäuseteil 3 besitzt ein Sichtfenster 7, das vorteilhaft aus durchsichtigem Material besteht, so dass durch das Sichtfenster 7 eine im oberen Gehäuseteil 3 angeordnete Injektionshülse 17 sichtbar ist. Das Injektionsgerät 1 besitzt eine Längsmittelachse 50, die in Längsrichtung des Injektionsgeräts 1 verläuft.

Das distale Ende des Injektionsgeräts 1 ist das einer am Injektionsgerät gehaltenen Injektionsnadel 8 abgewandt liegende Ende. Mit "proximal" wird die Seite des Injektionsgeräts 1 bezeichnet, die bei einer Injektion der Einstichstelle zugewandt liegt, und mit "distal" die Seite, die der Einstichstelle abgewandt liegt. Die proximale Richtung bezeichnet die Injektionsrichtung, also die Richtung zur Injektionsnadel 8 hin bzw. die Richtung, in der die Injektionsflüssigkeit aus einem Behälter ausgepresst wird. Die distale Richtung bezeichnet die entgegengesetzte Richtung, also von der Injektionsnadel 8 weg.

Die Fig. 1 und 2 zeigen das Injektionsgerät 1 in einer Nullstellung 28, in der keine Dosis von Injektionsflüssigkeit eingestellt ist. Wie Fig. 2 zeigt, ist im Halter 4 ein Behälter 5 mit Injektionsflüssigkeit angeordnet. Im Behälter 5 ist ein Stopfen 10 angeordnet, an dem eine Kolbenscheibe 13 eines Dosierkolbens 11 anliegt. Der Dosierkolben 11 umfasst außerdem eine Kolbenstange 12, die ein Außengewinde 72 trägt.

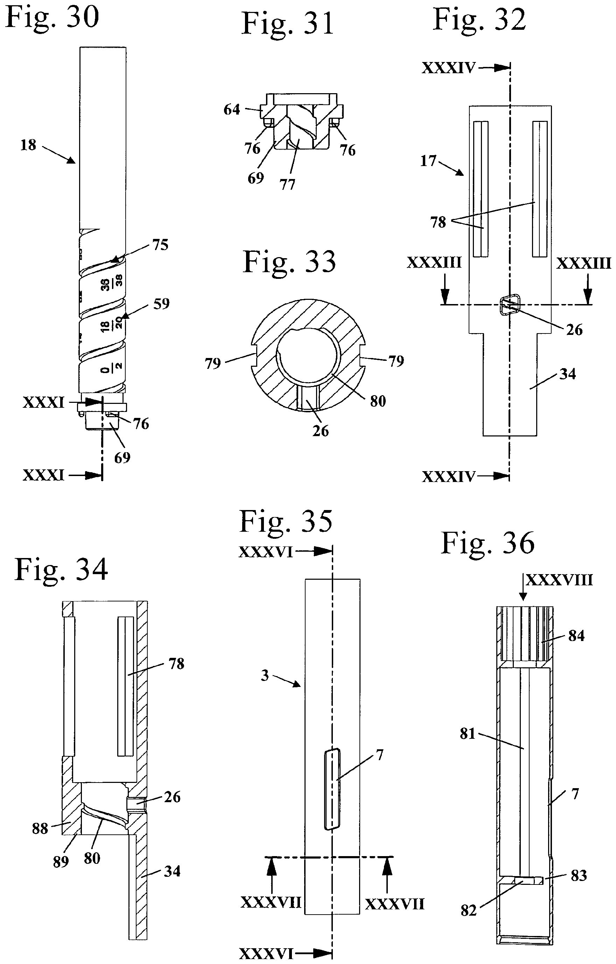

Die Außenseite der Injektionshülse 17 ist durch das Sichtfenster 7 des oberen Gehäuseteils 3 sichtbar. Die Injektionshülse 17 besitzt eine Öffnung 26, durch die der Außenumfang eines innerhalb der Injektionshülse 17 angeordneten Dosierorgans 18 sichtbar ist. Das Dosierorganl8, das auch als Skalenrohr bezeichnet werden kann, trägt an seinem Außenumfang eine in Fig. 30 gezeigte Skala 59, die durch das Sichtfenster 27 und die Öffnung 26 für den Bediener sichtbar ist und die eingestellte Dosis von auszupressender Injektionsflüssigkeit anzeigt.

Die Injektionshülse 17 ist im oberen Gehäuseteil 3 in Richtung der Längsmittelsachse 50 verschiebbar und gegenüber dem oberen Gehäuseteil 3 drehfest gehalten. Das Dosierorgan 18 und die Injektionshülse 17 sind über eine erste Gewindeverbindung 19 miteinander verbunden. Das Dosierorgan 18 ist an einem Drehlager 21 drehbar und axial unverschiebbar am oberen Gehäuseteil 3 gelagert. Das Dosierorgan 18 ist über eine zweite Gewindeverbindung 22 mit dem Außengewinde 72 der Kolbenstange 12 verbunden.

Im oberen Gehäuseteil 3 ist ein Mitnehmer 14 gelagert. Der Mitnehmer 14 ist über eine drehfeste Verbindung 24 drehfest mit dem Dosierorgan 18 verbunden. Die drehfeste Verbindung 24 kann eine Pressverbindung sein. Es kann jedoch auch vorgesehen sein, dass die drehfeste Verbindung 24 eine formschlüssige Verbindung ist. Der Mitnehmer 14 ist an einem im oberen Gehäuseteil 3 ausgebildeten Drehlager 15 drehbar und in Richtung der Längsmittelachse 50 ortsfest gelagert. Das Drehlager 15 wird durch einen Rand des oberen Gehäuseteils 3 gebildet. Im Rahmen der üblichen Fertigungstoleranzen kann der Mitnehmer 14 dabei in Richtung der Längsmittelachse 50 gegenüber dem oberen Gehäuseteil 3 beweglich sein.

Wie Fig. 2 auch zeigt, ist im oberen Gehäuseteil 3 eine Injektionsfeder 9 angeordnet, die als Schraubendruckfeder ausgebildet ist. Die Injektionsfeder 9 stützt sich mit einem ersten Ende 70 an einem Anlagerand 27 der Injektionshülse 17 ab und mit einem zweiten Ende 71 an einem Anlagerand 25 des oberen Gehäuseteils 3. Am Anlagerand 25 ist auch das Drehlager 15 für den Mitnehmer 14 ausgebildet.

Das Bedienelement 6 ist über ein Verbindungselement 56, das als Hülse ausgebildet ist, drehfest mit der Kolbenstange 12 verbunden. Das Bedienelement 6 stützt sich über eine Einstellfeder 23, die als Schraubendruckfeder ausgebildet ist, gegenüber dem oberen Gehäuseteil 3 ab. Die Einstellfeder 23 stützt sich dabei mit ihrem einen, distalen Ende am Bedienelement 6 und mit ihrem anderen, proximalen Ende am Rand 25 des oberen Gehäuseteils 3 ab. Die Einstellfeder 23, die das Bedienelement 6 in distale Richtung drückt, hat auf die Geschwindigkeit der Injektion keinen Einfluss. Die Einstellfeder 23 ist lediglich so ausgelegt, dass der Bediener das Bedienelement 6 mit angenehmer Kraft betätigen kann. Am Bedienelement 6 ist ein Absatz 32 ausgebildet, der bei in proximale Richtung gedrücktem Bedienelement 6 mit einem Rand 33 des oberen Gehäuseteils 3 zusammenwirkt und mit diesem einen Anschlag bildet, der die proximale Position des Bedienelements 6 begrenzt. Zwischen dem Bedienelement 6 und dem oberen Gehäuseteil 3 wirkt eine Rasteinrichtung 35, die mehrere Rastarme 36 umfasst, von denen in Fig. 2 einer sichtbar ist. Das Bedienelement 6 ist in der in den Fig. 1 und 2 gezeigten Nullstellung 28 über eine Kupplung 16 drehfest mit dem Mitnehmer 14 gekoppelt. Zwischen dem Bedienelement 6 und dem Mitnehmer 14 ist außerdem eine Einstellvorrichtung 41 gebildet, die eine Vielzahl von Rastvertiefungen 44 im Mitnehmer 14 umfasst.

Bei unbetätigtem Bedienelement 6 drückt die Einstellfeder 23 das Bedienelement 6 in seine distale Position, in der die Kupplung 20 geöffnet und das Bedienelement 6 gegenüber dem Gehäuse 2 drehbar ist. Zum Einstellen einer auszupressenden Menge an Injektionsflüssigkeit dreht der Bediener das Bedienelement 6 um die Längsmittelachse 50. Dabei dreht sich der über die Kupplung 16 drehfest mit dem Bedienelement 6 verbundene Mitnehmer 14 mit. Der Mitnehmer 14 ist über eine drehfeste Verbindung 24 mit dem Dosierorgan 18 verbunden, das sich ebenfalls mitdreht. Die Kolbenstange 12 ist über das Verbindungselement 56 drehfest mit dem Bedienelement 6 verbunden und dreht sich ebenfalls mit. Aufgrund der ersten Gewindeverbindung 19 und der drehfesten Fixierung der Injektionshülse 17 im oberen Gehäuseteil 3 wird die Injektionshülse 17 bei der Drehbewegung des Dosierorgans 18 in distale Richtung 30 bewegt. Dabei bewegt sich der Anlagerand 27 auf den Anlagerand 25 der Injektionsfeder 9 zu, wodurch die Injektionsfeder 9 gespannt wird.

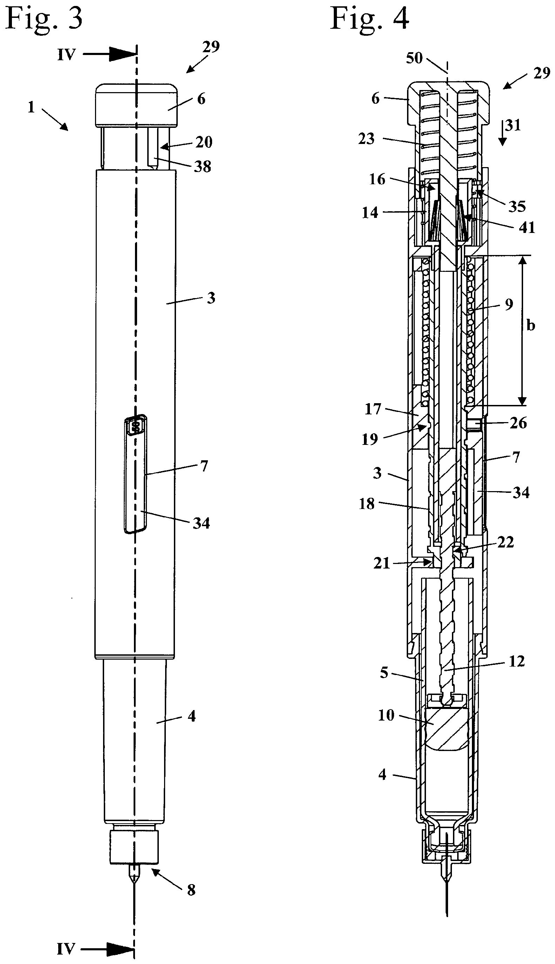

Die Fig. 3 und 4 zeigen das Injektionsgerät 1 in einer Maximalstellung 29, in der die maximale Dosis eingestellt ist. Die Länge der Injektionsfeder 9 hat sich von der in Fig. 2 gezeigten, weitgehend entspannten Länge a auf die in Fig. 4 gezeigte gespannte Länge b verkürzt. Auch in Nullstellung 28 ist die Injektionsfeder 9 zumindest geringfügig vorgespannt, so dass die Injektionsflüssigkeit vollständig ausgepresst werden kann. Wie Fig. 3 zeigt, ist durch das Sichtfenster 7 in Maximalstellung 29 die maximale Dosis sichtbar. Die Injektionshülse 17 besitzt einen Steg 34, der in proximale Richtung ragt und den durch das Sichtfenster 7 sichtbaren Bereich des Dosierorgans 18, der nicht die eingestellte Dosis anzeigt, abdeckt.

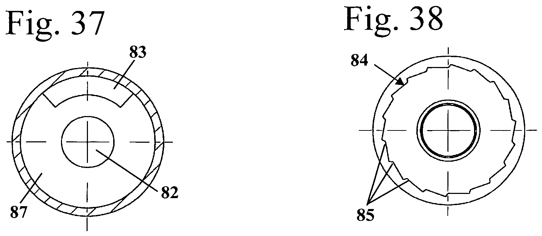

Zum Auspressen einer eingestellten Menge an Injektionsflüssigkeit drückt der Bediener das Bedienelement 6 entgegen der Kraft der Einstellfeder 23 in proximale Richtung 31. Dadurch gelangen Stege 38 der Kupplung 20 (Fig. 1) in Eingriff mit Rastelementen 85 der Rasteinrichtung 35 (Fig. 38). Dadurch wird das Bedienelement 6 gegenüber dem oberen Gehäuseteil 3 drehfest fixiert. Gleichzeitig wird die Kupplung 16 aufgrund der axialen Relativbewegung des Bedienelements 6 gegenüber dem Mitnehmer 14 mindestens teilweise gelöst, so dass sich der Mitnehmer 14 gemeinsam mit dem Dosierorgan 18 um die Längsmittelachse 50 drehen kann. Die Drehbewegung erfolgt aufgrund der von der gespannten Injektionsfeder 9 auf die Injektionshülse 17 ausgeübten axialen Kraft, die eine Drehung des Dosierorgans 18 bewirkt. Die Drehung erfolgt aufgrund der Gewindeverbindung 19 und der drehfesten Führung der Injektionshülse 17 im Gehäuseteil 3. Die Kolbenstange 12 ist über das Verbindungselement 56 und das Bedienelement 6 drehfest mit dem oberen Gehäuseteil 3 verbunden. Die zweite Gewindeverbindung 22 bewirkt bei der Drehung des Dosierorgans 18 deshalb eine Bewegung der Kolbenstange 12 in proximaler Richtung 31. Dadurch wird die eingestellte Menge an Injektionsflüssigkeit ausgepresst.

Die Injektion erfolgt nach dem Lösen der Kupplung 16 aufgrund der in der Injektionsfeder 9 gespeicherten Kraft automatisch. Zur Einstellung der Injektionsgeschwindigkeit besitzt das Injektionsgerät 1 die Einstellvorrichtung 41. Die Einstellvorrichtung 41 beeinflusst die Energie, nämlich das Drehmoment, das zur Drehung des Mitnehmers 14 gegenüber dem Bedienelement 6 benötigt wird. Das benötigte Drehmoment ist dabei abhängig von der axialen Position des Bedienelements 6 gegenüber dem oberen Gehäuseteil 3 und dem Mitnehmer 14. Dies wird im Folgenden noch näher erläutert.

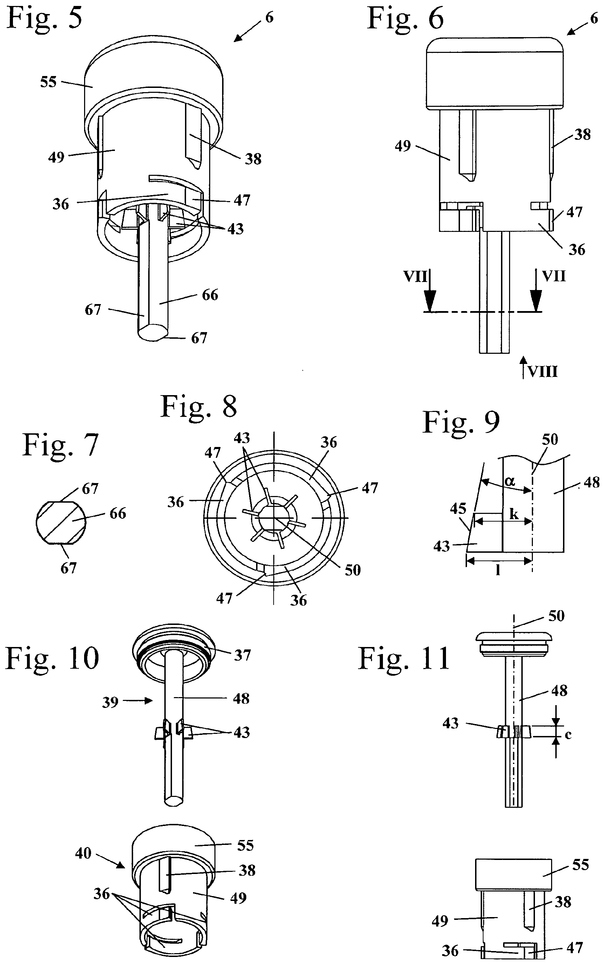

Die Fig. 5 bis 11 zeigen den Aufbau des Bedienelements 6 im Einzelnen. Das Bedienelement 6 besitzt einen Bedienabschnitt 55, der aus dem oberen Gehäuseteil 3 ragt und an dem der Bediener das Bedienelement 6 drehen oder in proximale Richtung 31 verschieben kann. Das Bedienelement 6 besitzt einen in den Fig. 5 und 6 gezeigten Hülsenabschnitt 49, der in Nullstellung 28 und Maximalstellung 29 teilweise aus dem oberen Gehäuseteil 32 ragt und an dem die Stege 38 der Kupplung 20 fixiert sind. An seinem proximalen Ende trägt der Hülsenabschnitt 49 im Ausführungsbeispiel insgesamt drei Rastarme 36, die an ihrem freien Ende jeweils ein Rastelement 47 tragen. Dies ist auch in Fig. 8 gezeigt. Die nach außen ragenden Rastelemente 47 sind mit den Rastarmen 36 radial nach innen beweglich gelagert.

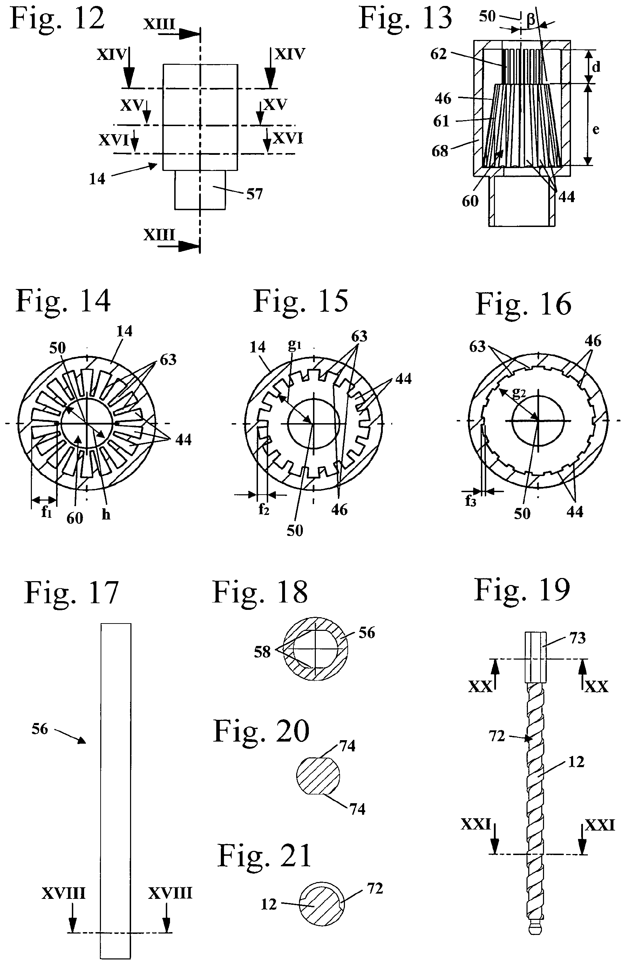

Wie Fig. 5 auch zeigt, besitzt das Bedienelement 6 einen Verbindungsstutzen 66, der an seinem Außenumfang zwei Abflachungen 67 zur drehfesten Verbindung mit dem Verbindungselement 56 trägt. Wie die Fig. 17 und 18 zeigen, besitzt das Verbindungselement 56, das hülsenförmig ausgebildet ist, an seiner Innenseite entsprechende Abflachungen 58, die mit den Abflachungen 67 des Verbindungsstutzens 66 zusammenwirken und das Bedienelement 6 und das Verbindungselement 56 dadurch drehfest miteinander verbinden. Wie die Fig. 19 und 20 zeigen, trägt die Kolbenstange 12 an einem distalen Endabschnitt 73 entsprechende Abflachungen 74 zur drehfesten Verbindung von Kolbenstange 12 und Verbindungselement 56. In den Fig. 19 und 21 ist auch das Außengewinde 72 der Kolbenstange 12 gezeigt.

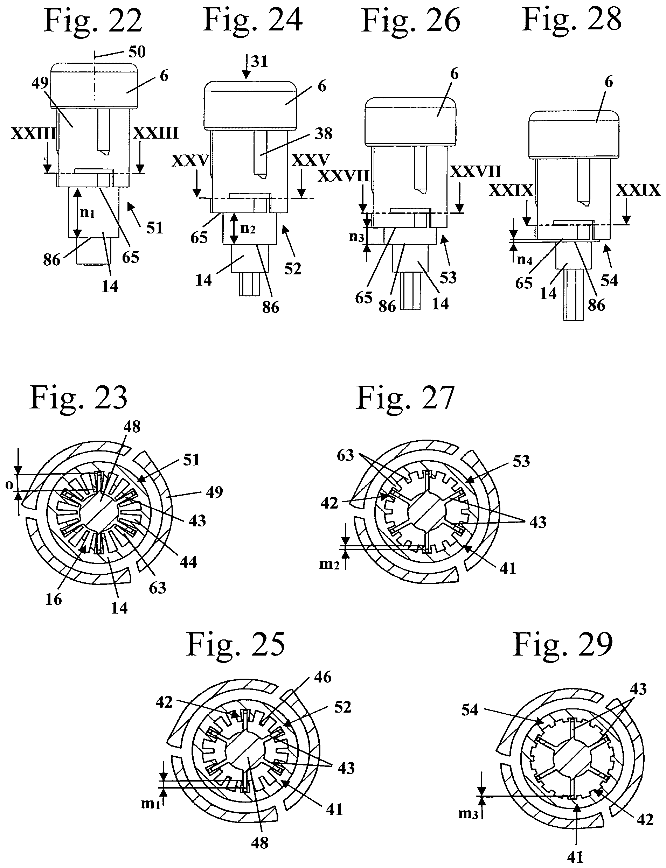

Wie die Fig. 5 und 8 zeigen, besitzt das Bedienelement 6 im Ausführungsbeispiel sechs Raststege 43, die mit den Rastvertiefungen 44 des Mitnehmers 14 eine Rasteinrichtung 42 (Fig. 25) bilden. Die Raststege 43 sind im Ausführungsbeispiel als bezogen auf die Längsmittelachse 50 radial nach außen ragende Rippen mit etwa konstanter Dicke ausgebildet. Die Dicke der Raststege 43 ist dabei deutlich geringer als die radiale Erstreckung. Wie Fig. 9 schematisch zeigt, besitzt jeder Raststeg 43 eine radial außen liegende Rastkante 45. Die Rastkanten 45 sind gegenüber der Längsmittelachse 50 um einen Winkel α geneigt. In der gezeigten Ausführung beträgt der Winkel α von 1° bis 20°, insbesondere von 2° bis 10°. Der Winkel α ist auf den gewünschten Betätigungsweg zwischen der langsamsten und der schnellsten einstellbaren Injektionsgeschwindigkeit und die gewünschte Differenz zwischen der langsamsten und der schnellsten Injektionsgeschwindigkeit abgestimmt. An seiner proximalen Seite besitzt der Raststeg 43 einen Abstand l zur Längsmittelachse 50, der deutlich größer als der Abstand k der Rastkante 45 an der distalen Seite des Raststegs 43 ist. Die Differenz zwischen den Abständen k und l ist in Abhängigkeit der gewünschten Auslegung geeignet gewählt.

Wie Fig. 9 schematisch zeigt, sind die Raststege 43 an einem Stiftabschnitt 48 des Bedienelements 6 angeordnet, der innerhalb des Hülsenabschnitts 49 und mit radialem Abstand zum Hülsenabschnitt 49 verläuft. An den Stiftabschnitt 48 schließt am proximalen Ende der Verbindungsstutzen 66 an. Im Ausführungsbeispiel sind die Raststege 43 einteilig mit dem Bedienelement 6 ausgebildet und bestehen aus dem gleichen Material wie das Bedienelement 6. Es kann jedoch auch vorteilhaft sein, zur Einstellung einer gewünschten Rastcharakteristik die Raststege 43 aus einem anderen Material auszubilden, beispielsweise aus einem Elastomer oder Gummi.

Wie Fig. 10 und 11 zeigen, ist das Bedienelement 6 aus einem ersten Einzelteil 39 und einem zweiten Einzelteil 40 aufgebaut, um die Fertigung und Montage zu vereinfachen. Das Bedienelement 6 kann auch aus einer größeren Anzahl von Einzelteilen gebildet sein. Die Einzelteile 39 und 40 sind im Ausführungsbeispiel an einem Rastrand 37 des ersten Einzelteils 39 fest miteinander verbunden.

Die Fig. 12 bis 16 zeigen die Gestaltung des Mitnehmers 14 im Einzelnen. Der Mitnehmer 14 besitzt einen Lagerabschnitt 57 mit verringertem Durchmesser, mit dem der Mitnehmer 14 drehbar im oberen Gehäuseteil 3 gelagert ist. Wie Fig. 13 zeigt, besitzt der Mitnehmer 14 in seinem Inneren einen im proximalen Bereich angeordneten konischen Abschnitt 61, an dessen distaler Seite sich ein zylindrischer Abschnitt 62 anschließt. Der konische Abschnitt 61 und der zylindrische Abschnitt 62 werden durch eine Vielzahl von in den Fig. 14 bis 16 gezeigten Stegen 63 gebildet, die von einer zylindrischen Außenwand 68 des Mitnehmers 14 radial nach innen ragen. In Umfangsrichtung zwischen den Stegen 63 sind die Rastvertiefungen 44 gebildet. Die Stege 63 besitzen radial innen liegende Gegenrastkanten 46, die gegenüber der Längsmittelachse 50 um einen Winkel β geneigt sind. Der Winkel β öffnet dabei in proximaler Richtung. Der Winkel β ist insbesondere gleich groß wie der Winkel α der Rastkanten 45 der Raststege 43. In montiertem Zustand des Injektionsgeräts 1 befinden sich die Raststege 43 innerhalb des Mitnehmers 14, im Ausführungsbeispiel innerhalb des von der zylindrischen Außenwand 68 umschlossenen Raums.

Wie Fig. 11 zeigt, besitzen die Raststege 43 eine in Richtung der Längsmittelachse 50 gemessene Höhe c. Der zylindrische Abschnitt 62 besitzt eine Höhe d, die im Ausführungsbeispiel geringfügig größer als die Höhe c ist. Im zylindrischen Abschnitt 62 begrenzen die Stege 63 einen Innenraum 60, dessen in Fig. 14 gezeigter Innendurchmesser h nur geringfügig größer als der Außendurchmesser des Stiftabschnitts 48 ist. Die Stege 63 besitzen im zylindrischen Abschnitt 62 eine radial gemessene Höhe f₁, die etwa der radialen Erstreckung der Raststege 43 entspricht.

Fig. 15 zeigt einen Schnitt durch den konischen Abschnitt 61. In der in Fig. 15 gezeigten Schnittebene besitzen die radial innen liegenden Rastkanten 46 der Stege 63 zur Längsmittelachse 50 einen Abstand g₁, der kleiner als der Abstand l der Rastkante 45 zur Längsmittelachse 50 (Fig. 9) ist. Befindet sich die proximale Seite der Raststege 43 in der in Fig. 15 gezeigten Schnittebene, so ragen die Raststege 43 in die Rastvertiefungen 44. Die radial gemessene Höhe f₂ der Stege 63 ist in der in Fig. 15 gezeigten Schnittebene durch den konischen Abschnitt 61 deutlich kleiner als die Höhe f₁ im zylindrischen Abschnitt 62.

Fig. 16 zeigt einen Schnitt durch den konischen Abschnitt 61 benachbart zum proximalen Ende des Innenraums 60. In dieser Schnittebene besitzen die Rastkanten 46 zur Längsmittelachse 50 einen Abstand g₂, der deutlich größer als der Abstand g₁ ist. Der Abstand g₂ kann näherungsweise dem Abstand l der Rastkante 45 zur Längsmittelachse 50 entsprechen. Befindet sich die proximale Seite der Raststege 43 in der in Fig. 16 gezeigten Schnittebene, so greifen die Raststege 43 nicht oder nur gering zwischen die Stege 63 ein. Die radial gemessene Höhe f₃ der Stege 63 ist in der in Fig. 15 gezeigten Schnittebene durch den konischen Abschnitt 61 deutlich kleiner als die Höhe f₂ in der in Fig. 15 gezeigten, distal liegenden Schnittebene.

Die Fig. 22 bis 29 zeigen Bedienelement 6 und Mitnehmer 14 in unterschiedlichen axialen Relativpositionen. Die Fig. 22 und 23 zeigen Bedienelement 6 und Mitnehmer 14 in einer Kupplungsposition 51. In dieser Position sind das Bedienelement 6 und der Mitnehmer 14 über die Kupplung 16 drehfest miteinander verbunden. Das Bedienelement 6 ist von der Einstellfeder 23 in diese Position vorgespannt. Die Kupplung 16 wird durch die Stege 63 im zylindrischen Abschnitt 62 gebildet. Die Stege 63 ragen bis nahe an den Stiftabschnitt 48 und überlappen die Stege 63 in Umfangsrichtung an ihrer distalen Seite mit einer Eingriffstiefe o. Die Eingriffstiefe o ist so gewählt, dass Mitnehmer 14 und Bedienelement 6 drehfest miteinander verbunden sind. Die Fig. 23, 25, 27 und 29 zeigen dabei Schnitte durch das Bedienelement 6 an der distalen Seite der Raststege 43. Der Hülsenabschnitt 49 besitzt eine proximale Stirnseite 65. Die proximale Stirnseite 65 besitzt in der Kupplungsposition 51 zu einer Unterkante 86 des Mitnehmers 14 einen in Richtung der Längsmittelachse 50 gemessenen ersten Abstand n₁. Die Unterkante 86 ist dabei die Kante des Mitnehmers 14, die an dem nach innen ragenden Rand des oberen Gehäuseteils 3 aufliegt.

Die Fig. 24 und 25 zeigen Bedienelement 6 und Mitnehmer 14 in einer ersten Relativposition 52, in der die Einstellvorrichtung 41 zwischen dem Bedienelement 6 und dem Mitnehmer 14 wirkt. Die proximale Stirnseite 65 des Hülsenabschnitts 49 besitzt zur Unterkante 86 des Mitnehmers 14 einen Abstand n₂, der kleiner ist als der Abstand n₁.

Wie Fig. 25 zeigt, befinden sich die Raststege 43 im konischen Abschnitt 61, in dem der Abstand der Rastkanten 46 der Stege 63 zum Stiftabschnitt 48 und zur Längsmittelachse 50 verringert ist. In dieser Relativposition überlappen die Raststege 43 die Stege 63 in radialer Richtung an der distalen Seite der Raststege 43 mit einer Rasttiefe m₁. Die Rasttiefe m₁ ist deutlich kleiner als die Eingriffstiefe o in der Kupplungsposition 51. Die Rasttiefe m₁ ist so gewählt, dass sich der Mitnehmer 14 gegenüber dem Bedienelement 6 unter Verformung der Raststege 43 drehen kann.

Um aus der Kupplungsposition 51 in die erste Relativposition 52 zu gelangen, muss der Bediener das Bedienelement 6 in proximaler Richtung 31 entgegen der Kraft der Einstellfeder 23 bewegen, wie in Fig. 24 angedeutet. Dadurch verschiebt sich das Bedienelement 6 relativ zum oberen Gehäuseteil 3 und die Stege 38 am Bedienelement 6 gelangen in Eingriff mit einer in den Fig. 36 und 38 gezeigten Verrastung 84 an der Innenseite des oberen Gehäuseteils 3. Die Verrastung 84 besitzt eine Vielzahl von Rastelementen 85, die das Bedienelement 6 drehfest gegenüber dem äußeren Gehäuseteil 3 sichern. Die Stege 38 bilden mit der Verrastung 84 die Kupplung 20.

Bei der in den Fig. 24 und 25 gezeigten ersten Relativposition 52 kann sich der Mitnehmer 14 relativ zum oberen Gehäuseteil 3 und zum Bedienelement 6 drehen, wenn die in der Injektionsfeder 9 gespeicherte Energie ausreichend ist, um die Raststege 43 zu verformen sowie den Stopfen 10 in proximale Richtung zu schieben, so dass die Injektionsflüssigkeit aus dem Behälter 5 ausgepresst wird. Das Auspressen von Injektionsflüssigkeit erfolgt jedoch aufgrund des hohen Drehmoments, das für eine Drehung des Mitnehmers 14 gegenüber dem Bedienelement 6 benötigt wird, sehr langsam. Die Energie, die zur Drehung des Mitnehmers 14 gegenüber dem Bedienelement 6 benötigt wird, ist groß.

Bei der in den Fig. 26 und 27 gezeigten zweiten Relativposition 53 von Bedienelement 6 und Mitnehmer 14 hat der Bediener das Bedienelement 6 weiter entgegen der Kraft der Einstellfeder 23 in proximaler Richtung 31 in das obere Gehäuseteil 3 gedrückt. Die proximale Stirnseite 65 besitzt in dieser Position einen dritten Abstand m₃ zur Unterkante 86 des Mitnehmers 14. Der dritte Abstand m₃ ist deutlich kleiner als der zweite Abstand m₂. Beim Bewegen des Bedienelements 6 in proximale Richtung 31 haben sich die Raststege 43 im konischen Abschnitt 61 weiter in proximale Richtung, also in Richtung auf einen vergrößerten Innendurchmesser des konischen Abschnitts 61 bewegt. Die distale Seite der Raststege 43 besitzt in radialer Richtung zur Längsmittelachse 50 eine geringe Überlappung zu den Stegen 63, so dass sich nur eine geringe Rasttiefe m₂ ergibt. In dieser Position der Einstellvorrichtung 41 ist das für eine Drehung des Mitnehmers 14 gegenüber dem Bedienelement 6 notwendige Drehmoment deutlich kleiner als bei der in den Fig. 24 und 25 gezeigten ersten Relativposition 52. Die Energie, die zur Drehung des Mitnehmers 14 gegenüber dem Bedienelement 6 benötigt wird, ist geringer als in der ersten Relativposition 52. Die Raststege 43 müssen nur geringfügig verformt werden, um die Stege 63 zu überwinden und in die nächste Rastposition zu gelangen. Dadurch erfolgt eine Injektion in der in Fig. 26 und 27 gezeigten zweiten Relativposition mit höherer Geschwindigkeit als bei der in Fig. 24 und 25 gezeigten ersten Relativposition.

Die Fig. 28 und 29 zeigen Bedienelement 6 und Mitnehmer 14 in einer dritten Relativposition 54, in der die Unterkante 86 zur proximalen Stirnseite 65 nur einen sehr geringen vierten Abstand n₄ aufweist. Das Bedienelement 6 befindet sich in der dritten Relativposition 54 in seiner proximalen Endposition, in der der in Fig. 2 gezeigte Absatz 32 am Rand 33 des oberen Gehäuseteils 3 anliegt. Wie Fig. 29 zeigt, besitzen die Raststege 43 in der dritten Relativposition 54 eine äußerst geringe Überlappung in radialer Richtung zu den Stegen 63. Die Rasttiefe m₃ ist minimal. Es kann auch vorgesehen sein, dass die Rasttiefe m₃ null ist, so dass die Raststege 43 sich gegenüber den Stegen 63 frei drehen können und die Einstellvorrichtung 41 in der dritten Relativposition 54 die Drehung des Mitnehmers 14 gegenüber dem Bedienelement 6 nicht bremst. In der dritten Relativposition 54 ergibt sich deshalb die größte mögliche Injektionsgeschwindigkeit. Die Energie, die zur Drehung des Mitnehmers 14 gegenüber dem Bedienelement 6 benötigt wird, ist gering. Die Injektionsgeschwindigkeit wird von der in der Injektionsfeder 9 gespeicherten Kraft und von den Reibkräften, die zwischen den zueinander bewegten Bauteilen wirken, bestimmt.

Die Einstellfeder 23 wirkt beim Injektionsvorgang in distaler Richtung auf das Bedienelement 6. Um die Injektionsgeschwindigkeit zu halten, muss der Bediener permanent auf das Bedienelement 6 drücken. Verringert oder verstärkt der Bediener den Druck auf das Bedienelement 6, so bewegt sich das Bedienelement 6 entsprechend in Richtung der Längsmittelachse 50, und die Injektionsgeschwindigkeit ändert sich. Dadurch ist eine dynamische Einstellung der Injektionsgeschwindigkeit während des Injektionsvorgangs möglich. Wird das Bedienelement 6 losgelassen, so stellt die Einstellfeder 23 das Bedienelement 6 in die Kupplungsposition 51 zurück, und der Injektionsvorgang wird angehalten. Durch erneutes Drücken des Bedienelements 6 kann der Injektionsvorgang fortgesetzt werden.

Im Ausführungsbeispiel sind der Winkel α der Rastkante 45 des Raststegs 43 und der Winkel β der Rastkante 46 des Stegs 63 gleich. Dadurch ergibt sich über die gesamte Höhe c der Raststege 43 die gleiche Rasttiefe m₁, m₂, m₃. Bei unterschiedlichen Winkeln α, β ergeben sich unterschiedliche Rasttiefen m in unterschiedlichen Abschnitten des Raststegs 43. Die von der Einstellvorrichtung 41 ausgeübte Kraft kann durch geeignete Wahl der Gestalt und Anzahl der Raststege 43 beeinflusst werden.

Die Fig. 30 und 31 zeigen das Dosierorgan 18 im Einzelnen. Das Dosierorgan 18 besitzt ein Außengewinde 75, das mit einem in Fig. 34 gezeigten Innengewinde 80 der Injektionshülse 17 die erste Gewindeverbindung 19 bildet. Das Dosierorgan 18 besitzt einen Lagerstutzen 69, mit dem das Dosierorgan 18 in einer Lageröffnung 82 im oberen Gehäuseteil 3 (Fig. 36 und 37) drehbar gelagert ist. Das Dosierorgan 18 besitzt Anlagestege 76, die an der distalen Seite der Gehäusewand 87, die die Lageröffnung 82 aufweist, anliegen. Dadurch wird die Reibung zwischen dem Dosierorgan 18 und dem oberen Gehäuseteil 3 bei der Drehbewegung des Dosierorgans 18 verringert. Die Anlagestege 76 sind an einem vom Lagerstutzen 69 nach außen ragenden Rand 64 angeordnet. Die distale Seite des Rands 64 bildet einen Anschlag für die proximale Endlage der Injektionshülse 17 und begrenzt damit die auszupressende Menge an Injektionsflüssigkeit. Alternativ kann der Anschlag für die proximale Endlage der Injektionshülse 17 auch an der distalen Fläche der Gehäusewand 87 (Fig. 37) des oberen Gehäuseteils 3 gebildet sein. Auch eine andere Gestaltung des Anschlags kann vorteilhaft sein. Wie Fig. 31 zeigt, ist im Lagerstutzen 69 ein Innengewinde 77 ausgebildet, das mit dem Außengewinde 72 der Kolbenstange 12 die zweite Gewindeverbindung 22 bildet (Fig. 2).

Wie Fig. 32 zeigt, besitzt die Injektionshülse 17 Aussparungen 78, die zur Gewichtsreduzierung dienen. In den Fig. 32 bis 34 ist auch die Öffnung 26 gezeigt, hinter der die Skala 59 des Dosierorgans 18 (Fig. 30) für den Bediener sichtbar ist.

Die Injektionshülse 17 besitzt an ihrem Außenumfang zwei einander gegenüberliegend angeordnete Führungsnuten 79. Im oberen Gehäuseteil 3 sind entsprechende Führungsstege 81 ausgebildet, von denen in Fig. 36 einer sichtbar ist. Die Führungsstege 81 ragen in die Führungsnuten 79 und führen dadurch die Injektionshülse 17 im oberen Gehäuseteil 3 in Richtung der Längsachse 50 beweglich und drehfest. Der das Innengewinde 80 aufweisende Abschnitt 88 der Injektionshülse 17 besitzt eine proximale Stirnseite 89, die in der proximalen Endlage der Injektionshülse 17 am Rand 64 des Dosierorgans 18 anliegt und mit diesem einen Anschlag für die proximale Endlage der Injektionshülse 17 bildet.

Wie Fig. 37 zeigt, weist die Gehäusewand 87 neben der Lageröffnung 82 eine Durchtrittsöffnung 83 auf, durch die der Steg 34 der Injektionshülse 17 ragt. Durch den Steg 34 wird eine geringe Baulänge des Injektionsgeräts 1 bei ausreichend großem Verstellbereich für das Dosierorgan 18 erreicht. Beim Einstellen der Dosis dreht sich das Dosierorgan 18, und die Injektionshülse 17 wird in axialer Richtung verschoben. Dadurch ist durch die Öffnung 26 jeweils die eingestellte Dosis sichtbar.

Wie Fig. 38 zeigt, sind die Rastelemente 85 der Verrastung 84 im Ausführungsbeispiel unsymmetrisch ausgebildet. Die Rastelemente 85 wirken mit den Rastelementen 47 am Bedienelement 6 (Fig. 5) zusammen. Aufgrund der unsymmetrischen Ausbildung der Rastelemente kann eine einmal eingestellte Dosis nicht verringert werden. Durch entsprechende Gestaltung der Rastelemente 85 und Rastelemente 47 kann jedoch auch ein Zurückdrehen des Bedienelements 6 zur Verringerung einer eingestellten Dosis möglich sein.

Über die Rastelemente 47 und 85 sind beim Einstellen einer auszupressenden Menge an Injektionsflüssigkeit Klicks hörbar. Da beim Auspressen einer Menge an Injektionsflüssigkeit das Bedienelement 6 und das obere Gehäuseteil 3 drehfest miteinander verbunden sind, sind die Klicks der Rasteinrichtung 35 beim Auspressen von Injektionsflüssigkeit nicht hörbar. Stattdessen sind beim Auspressen von Injektionsflüssigkeit Klicks der Rasteinrichtung 42 der Einstellvorrichtung 41 für den Bediener hörbar. Dabei erzeugt die Rasteinrichtung 42 in der ersten Rastposition 52 lautere Klicks mit größerem zeitlichem Abstand. Je weiter das Bedienelement 6 in proximale Richtung 31 gedrückt wird, umso leiser werden die Klicks und umso schneller folgen die Klicks aufeinander. Dadurch ist für den Bediener die Injektionsgeschwindigkeit hörbar. Die Rastelemente 85 bilden beim Einstellen der auszupressenden Dosis an Injektionsflüssigkeit mit den Rastelementen 47 die Rasteinrichtung 35. Beim Auspressen von Injektionsflüssigkeit wirken die Rastelemente 85 mit den Stegen 38 zusammen und bilden mit diesen die Kupplung 20 und verbinden das Bedienelement 6 drehfest mit dem oberen Gehäuseteil 3.

Für die Einstellvorrichtung 41 kann auch eine andere konstruktive Gestaltung zweckmäßig sein. Durch den konischen Abschnitt 61 ist mit der Einstellvorrichtung 41 eine stufenlose Einstellung der Injektionsgeschwindigkeit möglich. Es kann jedoch auch zweckmäßig sein, für die Einstellung der Injektionsgeschwindigkeit unterschiedliche Stufen vorzugeben, beispielsweise durch entsprechend gestuften Verlauf der Rastkante 46. Es kann vorgesehen sein, für die einzelnen einzustellenden Injektionsgeschwindigkeiten Raststellungen für das Bedienelement 6 vorzugeben. Die gezeigte Einstellvorrichtung 41 kann auch für Injektionsgeräte mit anderem konstruktiven Aufbau vorgesehen sein, die eine automatische Injektion vorsehen.

## Patentansprüche

1. Injektionsgerät zum automatischen Auspressen einer Dosis von Injektionsflüssigkeit aus einem Behälter, mit einem Gehäuse (2), das eine Längsmittelachse (50) besitzt, mit einem ersten Bauteil (6) und mit einem zweiten Bauteil (14), wobei sich das erste Bauteil und das zweite Bauteil beim Auspressen von Injektionsflüssigkeit relativ zueinander bewegen, und mit einer Injektionsfeder (9), wobei eine in der Injektionsfeder (9) gespeicherte Energie beim Auspressen von Injektionsflüssigkeit aus dem Behälter (5) mindestens teilweise freigegeben wird und das Auspressen der Dosis von Injektionsflüssigkeit aus dem Behälter (5) bewirkt, wobei das Injektionsgerät (1) eine Einstellvorrichtung (41) zur Einstellung der Injektionsgeschwindigkeit, mit der die Injektionsflüssigkeit aus dem Behälter (5) ausgepresst wird, besitzt, die die Energie, die zur Bewegung des zweiten Bauteils gegenüber dem ersten Bauteil benötigt wird, beeinflusst, wobei die Einstellvorrichtung (41) bei der Einstellung der Injektionsgeschwindigkeit auf die axiale Relativposition (52, 53, 54) von erstem Bauteil und zweitem Bauteil wirkt,
**dadurch gekennzeichnet, dass** das erste Bauteil beim Auspressen von Injektionsflüssigkeit in Richtung der Längsmittelachse (50) beweglich und drehfest im Gehäuse (2) geführt ist, dass das zweite Bauteil beim Auspressen von Injektionsflüssigkeit drehbar und in Richtung der Längsmittelachse (50) ortsfest gegenüber dem Gehäuse (2) gelagert ist und dass die Einstellvorrichtung (41) eine Rasteinrichtung (42) besitzt, die zwischen dem ersten Bauteil und dem zweiten Bauteil wirkt, wobei die Rasteinrichtung (42) in einer ersten Relativposition (52) von erstem Bauteil und zweitem Bauteil eine höhere Kraft zum Überwinden einer Rastung aufweist als in einer zweiten Relativposition (53) von erstem Bauteil und zweitem Bauteil.

2. Injektionsgerät nach Anspruch 1,
**dadurch gekennzeichnet, dass** die Einstellvorrichtung (41) eine stufenlose Einstellung der Injektionsgeschwindigkeit ermöglicht.

3. Injektionsgerät nach Anspruch 1 oder 2,
**dadurch gekennzeichnet, dass** die Einstellvorrichtung (41) ein Bedienelement (6) umfasst, das von einer Einstellfeder (23) in der entgegen einer Erhöhung der Injektionsgeschwindigkeit gerichteten Richtung vorgespannt ist.

4. Injektionsgerät nach Anspruch 3,
**dadurch gekennzeichnet, dass** das Bedienelement (6) das erste Bauteil bildet und zum Auspressen von Injektionsflüssigkeit entgegen der von der Einstellfeder (23) ausgeübten Kraft in proximaler Richtung (31) zu verschieben ist.

5. Injektionsgerät nach Anspruch 4,
**dadurch gekennzeichnet, dass** die Einstellfeder (23) zwischen dem Bedienelement (6) und dem Gehäuse (2) wirkt und sich insbesondere mit einem Ende am Bedienelement (6) und mit dem anderen Ende am Gehäuse (2) abstützt.

6. Injektionsgerät nach einem der Ansprüche 1 bis 5,
**dadurch gekennzeichnet, dass** die Rasteinrichtung (42) mindestens ein Rastelement umfasst, das mit mindestens einem Gegenrastelement zusammenwirkt, wobei die Rasttiefe (m₁, m₂, m₃), mit der die Rastelemente und die Gegenrastelemente sich überlappen, in der ersten Relativposition (52) größer als in der zweiten Relativposition (53) ist.

7. Injektionsgerät nach Anspruch 6,
**dadurch gekennzeichnet, dass** das Rastelement in einer Schnittdarstellung, die die Längsmittelachse (50) enthält, eine zur Längsmittelachse (50) geneigt verlaufende Rastkante (45) besitzt und dass das Gegenrastelement eine zur Längsmittelachse (50) geneigt verlaufende Gegenrastkante (46) besitzt.

8. Injektionsgerät nach einem der Ansprüche 6 oder 7,
**dadurch gekennzeichnet, dass** mindestens ein Rastelement der Rasteinrichtung (42) Teil einer Kupplung (16) zwischen dem ersten Bauteil und dem zweiten Bauteil ist, wobei die Kupplung (16) das erste Bauteil und das zweite Bauteil beim Einstellen einer auszupressenden Menge an Injektionsflüssigkeit drehfest verbindet.

9. Injektionsgerät nach einem der Ansprüche 6 bis 8,
**dadurch gekennzeichnet, dass** eine Vielzahl von Rastelementen und von Gegenrastelementen vorgesehen ist.

10. Injektionsgerät nach einem der Ansprüche 1 bis 9,
**dadurch gekennzeichnet, dass** das Injektionsgerät (1) eine Injektionshülse (17) besitzt, die sich beim Einstellen einer Dosis von Injektionsflüssigkeit in Richtung der Längsmittelachse (50) gegenüber dem Gehäuse (2) bewegt, und dass die Injektionsfeder (9) als Schraubendruckfeder ausgebildet ist und sich mit einem ersten Ende (70) gegenüber der Injektionshülse (17) und mit einem zweiten Ende (71) gegenüber dem Gehäuse (2) abstützt.

## Claims

1. An injection device for automatically squeezing out a dosage of injection liquid from a container, the injection device having a housing (2) which has a longitudinal central axis (50); having a first component (6) and a second component (14), wherein the first component and the second component move relative to one another on the squeezing out of injection liquid; and having an injection spring (9), wherein energy stored in the injection spring (9) is at least partially released on the squeezing out of injection liquid from the container (5) and causes the dosage of injection liquid to be squeezed out from the container (5), wherein the injection device (1) has a setting apparatus (41) to set the injection rate at which the injection liquid is squeezed out from the container (5), said setting apparatus (41) influencing the energy required for moving the second component in relation to the first component, wherein the setting apparatus (41), when setting the injection rate, acts on the axial relative position (53, 53, 54) of the first component and the second component,
**characterized in that** the first component, on the squeezing out of injection liquid, is guided in the housing (2) so as to be movable in the direction of the longitudinal central axis (50) and so as to be rotationally fixed; **in that** the second component, on the squeezing out of injection liquid, is supported in relation to the housing (2) so as to be rotatable and so as to be in a fixed position in the direction of the longitudinal central axis (50); and **in that** the setting apparatus (41) has a latching device (42) which acts between the first component and the second component, with the latching device (42) in a first relative position (52) of the first component and the second component having a greater force for overcoming a latching than in a second relative position (53) of the first component and the second component.

2. An injection device in accordance with claim 1,
**characterized in that** the setting apparatus (41) enables a stepless setting of the injection rate.

3. An injection device in accordance with claim 1 or claim 2,
**characterized in that** the setting apparatus (41) has an operating element (6) which is preloaded by a setting spring (23) in the direction opposite to an increase in the injection rate.

4. An injection device in accordance with claim 3,
**characterized in that** the operating element (6) forms the first component and, for squeezing out injection liquid, is to be displaced in the proximal direction (31), against the force exerted by the setting spring (23).

5. An injection device in accordance with claim 4,
**characterized in that** the setting spring (23) acts between the operating element (6) and the housing (2) and is in particular supported on the operating element (6) with one end and is supported on the housing (2) with the other end.

6. An injection device in accordance with any one of the claims 1 to 5,
**characterized in that** the latching device (42) comprises at least one latching element which cooperates with at least one counter latching element, with the latching depth (m₁, m₂, m₃), with which the latching elements and the counter latching elements overlap, being greater in the first relative position (52) than in the second relative position (53).

7. An injection device in accordance with claim 6,
**characterized in that** the latching element, in a sectional view which includes the longitudinal central axis (50), has a latching edge (45) which extends inclined to the longitudinal central axis (50); and **in that** the counter latching element has a counter latching edge (46) which extends inclined to the longitudinal central axis (50).

8. An injection device in accordance with one of the claims 6 or 7,
**characterized in that** at least one latching element of the latching device (42) is part of a coupling (16) between the first component and the second component, with the coupling (16) connecting the first component and the second component in a rotationally fixed manner on the setting of a quantity of injection liquid to be squeezed out.

9. An injection device in accordance with any one of the claims 6 to 8,
**characterized in that** a plurality of latching elements and counter latching elements are provided.

10. An injection device in accordance with any one of the claims 1 to 9,
**characterized in that** the injection device (1) has an injection sleeve (17) which, when setting a dosage of injection liquid, moves in relation to the housing (2) in the direction of the longitudinal central axis (50); and **in that** the injection spring (9) is configured as a compression coil spring and is supported in relation to the injection sleeve (17) with a first end and is supported in relation to the housing (2) with a second end.

## Revendications

1. Appareil d'injection pour expulser automatiquement une dose de liquide d'injection hors d'un récipient, comprenant un boîtier (2) ayant un axe central longitudinal (50), un premier composant (6) et un second composant (14), le premier composant et le second composant se déplaçant l'un par rapport à l'autre lors de l'expulsion du liquide d'injection, et comprenant un ressort d'injection (9),
dans lequel
une énergie stockée dans le ressort d'injection (9) est libérée au moins partiellement lors de l'expulsion du liquide d'injection hors du récipient (5) et provoque l'expulsion de la dose de liquide d'injection hors du récipient (5), et
l'appareil d'injection (1) possède un dispositif de réglage (41) pour régler la vitesse d'injection à laquelle le liquide d'injection est expulsé hors du récipient (5), dispositif qui influence l'énergie nécessaire pour déplacer le second composant par rapport au premier composant, et
lors du réglage de la vitesse d'injection, le dispositif de réglage (41) agit sur la position relative axiale (52, 53, 54) du premier composant et du second composant,
**caractérisé en ce que**
lors de l'expulsion du liquide d'injection, le premier composant est guidé dans le boîtier (2) de manière à pouvoir se déplacer dans la direction de l'axe central longitudinal (50) et de façon solidaire en rotation, et **en ce que** lors de l'expulsion du liquide d'injection, le second composant est monté de manière à pouvoir tourner et de façon stationnaire par rapport au boîtier (2) dans la direction de l'axe central longitudinal (50), et **en ce que** le dispositif de réglage (41) possède un moyen d'enclenchement (42) agissant entre le premier composant et le second composant, et
dans une première position relative (52) du premier composant et du second composant, le moyen d'enclenchement (42) présente une force pour surmonter un enclenchement, qui est plus élevée que celle dans une seconde position relative (53) du premier composant et du second composant.

2. Appareil d'injection selon la revendication 1,
**caractérisé en ce que**
le dispositif de réglage (41) permet un ajustement en continu de la vitesse d'injection.

3. Appareil d'injection selon la revendication 1 ou 2,
**caractérisé en ce que**
le dispositif de réglage (41) comprend un élément de commande (6) précontraint par un ressort de réglage (23) dans le sens opposé à une augmentation de la vitesse d'injection.

4. Appareil d'injection selon la revendication 3,
**caractérisé en ce que**
l'élément de commande (6) constitue le premier composant et doit être déplacé dans la direction proximale (31) à l'encontre de la force exercée par le ressort de réglage (23), pour expulser le liquide d'injection.

5. Appareil d'injection selon la revendication 4,
**caractérisé en ce que**
le ressort de réglage (23) agit entre l'élément de commande (6) et le boîtier (2) et s'appuie en particulier par une extrémité contre l'élément de commande (6) et par l'autre extrémité contre le boîtier (2).

6. Appareil d'injection selon l'une des revendications 1 à 5,
**caractérisé en ce que**
le moyen d'enclenchement (42) comprend au moins un élément d'enclenchement coopérant avec au moins un élément d'enclenchement antagoniste, et
la profondeur d'enclenchement (m₁, m₂, m₃) avec laquelle les éléments d'enclenchement et les éléments d'enclenchement antagonistes se chevauchent est plus grande dans la première position relative (52) que dans la seconde position relative (53).

7. Appareil d'injection selon la revendication 6,
**caractérisé en ce que**
en vue en coupe comprenant l'axe central longitudinal (50), l'élément d'enclenchement possède une arête d'enclenchement (45) s'étendant de manière inclinée par rapport à l'axe central longitudinal (50), et **en ce que** l'élément d'enclenchement antagoniste possède une arête d'enclenchement antagoniste (46) s'étendant de manière inclinée par rapport à l'axe central longitudinal (50).

8. Appareil d'injection selon l'une des revendications 6 ou 7,
**caractérisé en ce que**
au moins un élément d'enclenchement du moyen d'enclenchement (42) fait partie d'un couplage (16) entre le premier composant et le second composant, le couplage (16) reliant le premier composant et le second composant solidairement en rotation lors du réglage d'une quantité de liquide d'injection à expulser.

9. Appareil d'injection selon l'une des revendications 6 à 8,
**caractérisé en ce que**
il est prévu une pluralité d'éléments d'enclenchement et d'éléments d'enclenchement antagonistes.

10. Appareil d'injection selon l'une des revendications 1 à 9,
**caractérisé en ce que**
l'appareil d'injection (1) possède une douille d'injection (17) qui se déplace dans la direction de l'axe central longitudinal (50) lors du réglage d'une dose de liquide d'injection, et **en ce que**
le ressort d'injection (9) est réalisé sous forme de ressort de compression hélicoïdal et s'appuie par une première extrémité (70) contre la douille d'injection (17) et par une seconde extrémité (71) contre le boîtier (2).
